# EUROPÄISCHE PATENTSCHRIFT

(11) **EP 1 648 433 B1**
(45) Veröffentlichungstag und Bekanntmachung des Hinweises auf die Patenterteilung: **19.09.2007**
(21) Anmeldenummer: 04763387.0
(22) Anmeldetag: 22.07.2004
(51) Int. Cl.: A61K 31/135, A61K 31/34, A61K 31/381, A61K 31/40, A61K 31/4164, A61K 31/44, A61P 25/24

(54) **SUBSTITUIERTE 2-AMINOTETRALINE ZUR BEHANDLUNG VON DEPRESSIONEN**
SUBSTITUTED 2-AMINOTETRALINS FOR THE TREATMENT OF DEPRESSIONS
2-AMINOTETRALINES SUBSTITUÉES POUR LE TRAITEMENT DE DEPRESSIONS

(30) Priorität: 26.07.2003 DE 10334187
(43) Veröffentlichungstag der Anmeldung: 26.04.2006
(73) Patentinhaber: SCHWARZ PHARMA AG, 40789 Monheim (DE)
(72) Erfinder: SCHELLER, Dieter, 41470 Neuss (DE); BREIDENBACH, Alexander, 79576 Weil am Rhein (DE); SELVE, Norma, 53842 Troisdorf (DE)
(74) Vertreter: HOFFMANN EITLE
(86) Internationale Anmeldenummer: PCT/EP2004/008169
(87) Internationale Veröffentlichungsnummer: WO 2005/009425

(56) Entgegenhaltungen:
- EP-A- 0 334 538
- DE-A- 19 814 084
- US-A- 4 564 628
- US-A- 5 214 156
- BARTOSZYK G D: "ANXIOLYTIC EFFECTS OF DOPAMINE RECEPTOR LIGANDS: I. INVOLVEMENT OF DOPAMINE AUTORECEPTORS" LIFE SCIENCES, PERGAMON PRESS, OXFORD, GB, Bd. 62, Nr. 7, 1998, Seiten 649-663, XP001079854 ISSN: 0024-3205
- KOSTOWSKI W ET AL: "5-Hydroxytryptamine(1A) receptor agonists in animal models of depression and anxiety" PHARMACOLOGY AND TOXICOLOGY 1992 DENMARK, Bd. 71, Nr. 1, 1992, Seiten 24-30, XP008039045 ISSN: 0901-9928
- WELNER S A ET AL: "AUTORADIOGRAPHIC QUANTIFICATION OF SEROTONIN-1A RECEPTORS IN RAT BRAIN FOLLOWING ANTIDEPRESSANT DRUG TREATMENT" SYNAPSE (NEW YORK), Bd. 4, Nr. 4, 1989, Seiten 347-352, XP008039049 ISSN: 0887-4476
- TIMMERMAN, WIA ET AL: "The potential antipsychotic activity of the partial dopamine receptor agonist (+)N-0437" EUROPEAN JOURNAL OF PHARMACOLOGY , 181(3), 253-60 CODEN: EJPHAZ; ISSN: 0014-2999, 1990, XP008039005
- DRYER S E ET AL: "BIOCHEMICAL AND BEHAVIORAL ACTIONS OF 5 8 DI METHOXYLATED AMINO TETRALINS NEW NONOPIATE ANALGESIC AGENTS" FEDERATION PROCEEDINGS, Bd. 39, Nr. 3, 1980, Seite ABSTRACT 3051, XP008039043 & 64TH ANNUAL MEETING OF THE FED. AM. SOC. EXP. BIOL., ANAHEIM, CALIF., USA, APR. 13-18, 1980. FED PRO ISSN: 0014-9446
- PARK S ET AL: "Evaluation of an aminotetraline, CP 14.368, as an antidepressant." CURRENT THERAPEUTIC RESEARCH, CLINICAL AND EXPERIMENTAL. FEB 1972, Bd. 14, Nr. 2, Februar 1972 (1972-02), Seiten 65-70, XP008039025 ISSN: 0011-393X
- "SR 58611A SR 58611" DRUGS IN R AND D 2003 NEW ZEALAND, Bd. 4, Nr. 6, 2003, Seiten 380-382, XP008039046 ISSN: 1174-5886

## Beschreibung

Nach Schätzungen der WHO wird die Depression bis 2020 die zweithäufigste Ursache für erkrankungsbedingte Behinderung sein (Murray, Lancet 349 (1997) 1498). Die Effizienz gegenwärtiger pharmakologischer Behandlungen ist aus verschiedenen Gründen, z.B. wegen spätem Wirkeintritt, Nebenwirkungen oder fehlender Wirksamkeit der Arzneimittel begrenzt. Aufgrund der Häufigkeit und Dauer dieser Erkrankung und der Rezidivneigung besteht ein großer Bedarf an neuen, innovativen Antidepressiva.

Aus der US 4,564,628, der US 4,885,308, der US 4,722,933 und der WO 01/38321 sind substituierte 2-Aminotetraline bekannt. Es handelt sich um Substanzen mit dopaminerger Wirkung, die insbesondere zur Behandlung von Morbus Parkinson bekannt sind. Insbesondere das Rotigotin [(-)-5,6,7,8-Tetrahydro-6-[propyl[2-(2-thienyl)ethyl]amino]-1-naphthol] hat sich in klinischen Studien als effektives, transdermal verfügbares Antiparkinsonmittel erwiesen (Metman, Clinical Neuropharmacol. 24, 2001,163).

Es wurde nun überraschenderweise gefunden, dass besagte substituierte 2-Aminotetraline der allgemeinen Formel I 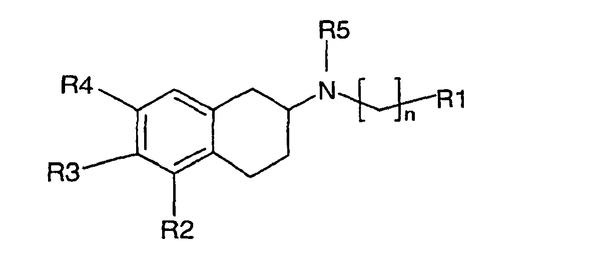 worin gilt:
n ist 1-5;
R2 ist OA; R3 und R4 sind jeweils unabhängig voneinander ausgewählt aus H und OA;
wobei A ausgewählt ist aus H, CI-3 Alkyl oder einer Gruppe 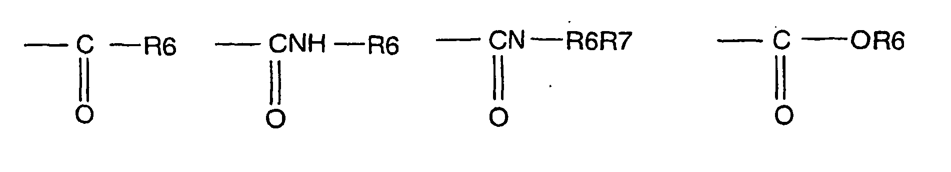 worin R6 und R7 jeweils unabhängig voneinander Alkyl, insbesondere C1-20 Alkyl, oder Aryl, insbesondere optional substituiertes Phenyl, sind;
R5 ein C1-3 Alkyl ist;
R1 eine Gruppe ist, die ausgewählt ist
worin X ausgewählt ist aus. S, O oder NH;
wobei die Verbindung der Formel I als Razemat oder als reines (R)- oder (S)-Enantiomer vorliegen kann,
sowie physiologisch akzeptable Salze dieser Verbindungen zur Herstellung von Arzneimitteln zur Behandlung von Depressionen nach Anspruch 1 geeignet sind.

Zur Herstellung eines Antidepressivums besonders geeignete Verbindungen sind solche, in denen R2 eine Gruppe OA ist und R3 und R4 unabhängig voneinander H oder eine Gruppe OA sind, wobei A besonders bevorzugt ausgewählt ist aus einem Wasserstoffatom oder einer Gruppe 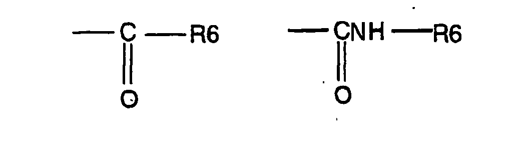 in der R6 ein C1-20 Alkyl, insbesondere C1-12 Alkyl, Phenyl oder Methoxyphenyl ist.

In einer anderen bevorzugten Ausführungsform der Erfindung ist R4 ein H.
In einer anderen bevorzugten Ausführungsform der Erfindung ist R3 ein H.
In einer anderen bevorzugten Ausführungsform der Erfindung sind R3 und R4 beide H.
In einer anderen bevorzugten Ausführungsform der Erfindung ist n= 1, 2 oder 3.

In einer anderen bevorzugten Ausführungsform der Erfindung sind R3 und R4 beide H und R2 ist -OH oder -O(CO)CH3, wobei n ganz besonders bevorzugt 2 ist.

R1 wind ausgewählt aus der Gruppe 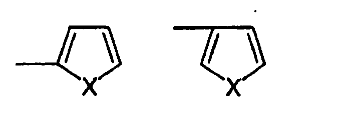 wobei X ausgewählt ist aus S, O und NH und wobei X ganz besonders bevorzugt ein Schwefelatom ist.

Ganz besonders bevorzugt ist R1 2-Thienyl.

In einer weiteren bevorzugten Ausführungsform der Erfindung stellt R5 ein C3-Alkyl dar.

In einer besonders bevorzugten Ausführungsform der Erfindung wird zur Herstellung des Arzneimittels zur Behandlung von Depressionen das Razemat von (+/-) 5,6,7,8-Tetrahydro-6-[propyl[2-(2-thienyl)ethyl]amino]-1-naphthol und ganz besonders bevorzugt das reine S-Enantiomer dieser Verbindung (Rotigotin) verwendet.

Unter den Begriffen "C1-20 Alkyl", "C1-12 Alkyl", "C1-3 Alkyl" werden jeweils verzweigte oder unverzweigte Alkylgruppen mit der entsprechenden Zahl C-Atome verstanden.
Beispielsweise umfasst ein "C1-20 Alkyl" alle Alkyle mit 1 bis 20 C-Atomen. Die Alkyle können optional substituiert sein, z.B. mit Halogen. Bevorzugt liegen die Alkyle unsubstituiert vor.

In drei verschiedenen, validierten Tiermodellen wurde die Eignung von Rotigotin als Antidepressivum demonstriert.

Der "forced swim test" ist ein Tiermodell bei dem depressive Episoden durch akuten Stress ausgelöst werden. Dabei werden Ratten in einem begrenzten Raum zum Schwimmen gezwungen. Nach initialen Selbstrettungsversuchen, in denen die Tiere die Ausweglosigkeit erfassen, verfallen sie in Bewegungslosigkeit. Bei einer Wiederholung des Versuchs verharren die Tiere von Beginn des Versuchs an in Bewegungslosigkeit. Bei Vorbehandlung mit Antidepressiva wird die Zeit der Bewegungslosigkeit beim Wiederholungsversuch verkürzt, die Tiere beginnen meist unmittelbar nach Transfer in das Wasserbecken mit Such- und Fluchtbewegungen (Porsolt, Biomedicine 30, 1979, 139). Rotigotin führt zu einer deutlich verkürzten Immobilitätszeit.

Im "learned helplessness test" werden Ratten mehrfach unkontrollierbarem Stress ausgesetzt. Dies bewirkt bei den Tieren eine verschlechterte Lernfähigkeit in einer späteren Situation (z.B. nach 48 h), in der sie dem Stress wieder ausweichen könnten. Nach subchronischer, aber nicht akuter Gabe von Antidepressiva normalisiert sich die Lernfähigkeit wieder und die Tiere lernen, dem (angekündigten) Stress (rechtzeitig) zu entfliehen. (Sherman, Pharmacology Biochemistry & Behavior 16, 1982, 449). Nach mehrtägiger Verabreichung von Rotigotindepotsuspensionen (Ausführungsbeispiel 2) zeigten die Tiere bei niedrigen Konzentrationen ein verbessertes Lernverhalten; allerdings steigerten die höheren Dosen auch die Aktivität der Tiere unter Nicht-Test-Bedingungen.

In einem weiteren Tiermodell (Ausführungsbeispiel 3) wurde untersucht, ob die antidepressiven Wirkungen von Rotigotin von einer allgemeinen motorischen Stimulation unterschieden werden können. Hierbei wurde Rotigotin an Ratten verabreicht, deren Riechkolben beidseitig entfernt wurden. Die Entfernung der Riechkolben führt in der unbehandelten Kontroll-Gruppe zu einer adaptiven Hyperaktivität. Es ist aus der Literatur bekannt, dass chronisch verabreichte Antidepressiva in diesem Modell zu einer Reduktion der Bewegungsaktivität der Tiere führen, während Stimulantien die motorische Aktivität weiter steigern (van Riezen H et al, Br J Pharmacol. 60(4), 1977, 521; Kelly JP et al, Pharmacol Ther. 74(3), 1997, 299). Mit diesem Modell kann somit zwischen antidepressiven und unspezifisch-stimulatorischen Effekten eines Wirkstoffs diskriminiert werden. Es zeigte sich, dass Rotigotin niedrig dosiert eine spezifisch antidepressive Wirkung zeigt, die in etwa der Wirkung des Antidepressivums Imipramin entspricht und die zur nahezu vollständigen Unterdrückung der Bulbektomie-induzierten lokomotorischen Hyperaktivität führt. Bei höheren Rotigotinkonzentrationen überwiegt hingegen der stimulatorische dopamin-agonistische Effekt.

Damit konnte klar gezeigt werden, dass subkutan appliziertes Rotigotin in allen drei Tests überraschenderweise eine signifikante antidepressive Wirkung hat.

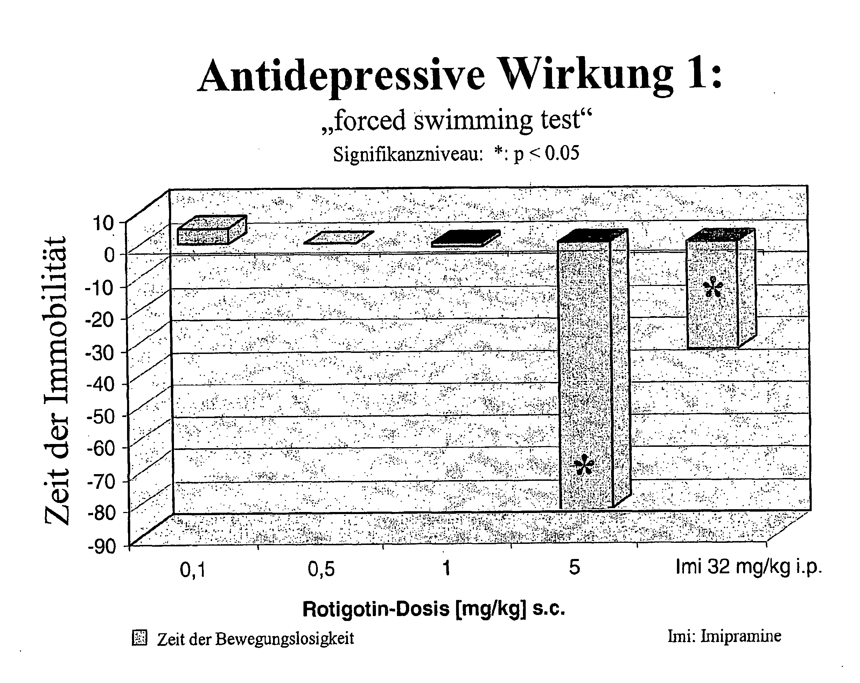

Abbildung 1 zeigt, dass Rotigotin im "forced swim test" zu einer deutlichen Reduktion der Immobilitätszeit führt.

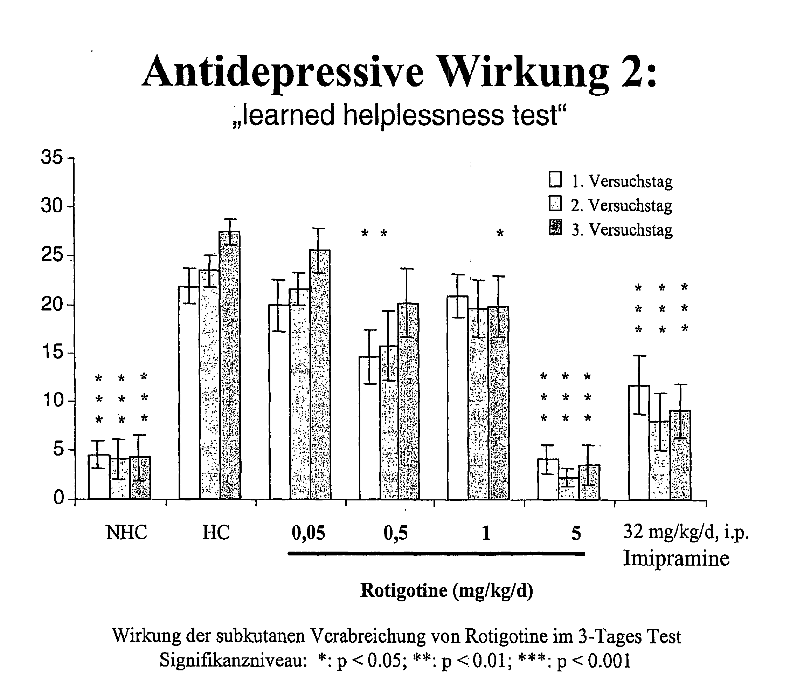

Abbildung 2 zeigt, dass mit Rotigotindepotsuspension (Ausführungsbeispiel 2) behandelte Tiere im "learned helplessness test" dosisabhängig ein normalisiertes Lernverhalten (NHC) gegenüber der nur mit Vehikel behandelten Kontrollgruppe (HC) zeigen.

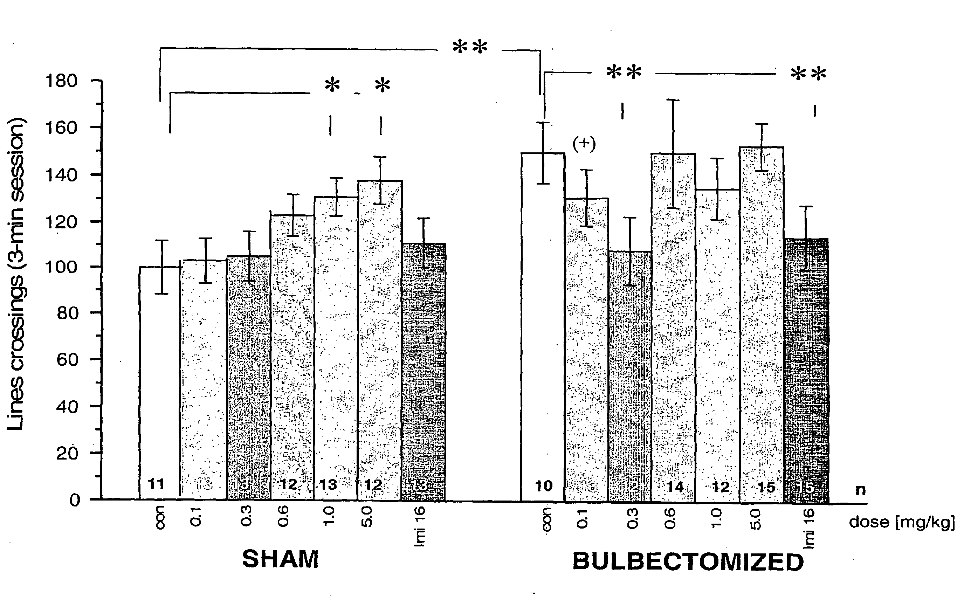

Abbildung 3 zeigt, dass Rotigotin in niedrigen Dosierungen in bulbectomisierten Ratten (Ausführungsbeispiel 3) die motorische Hyperaktivität deutlich reduziert und damit eine klare antidepressive Wirkung entfaltet. In höheren Dosierungen hingegen dominiert eine unspezifische Aktivierung der lokomotorischen Aktivität und tritt sowohl bei bulbectomisierten Tieren als auch bei Kontrolltieren auf.

Aus den präklinischen Daten ergibt sich die Schlussfolgerung, dass mit den als Antiparkinson-Wirkstoffen bekannten substituierten 2-Aminotetraline der allgemeinen Formel I neue wirksame Arzneimittel zur Behandlung von Depressionen zur Verfügung gestellt werden konnten.

Ein Gegenstand der Erfindung ist daher die Verwendung von Verbindungen der Formel 1, insbesondere Rotigotin sowie Salze dieser Verbindungen zur Herstellung eines Arzneimittels zur Behandlung von Depressionen. nach Anspruch 1.

Der Begriff "Behandlung" umfasst in dieser Patentanmeldung sowohl die Therapie bestehender Depressionen als auch die vorbeugende Therapie (Prophylaxe) von Depressionen, z.B. von rezidivierenden depressiven Phasen.

Depressive Störungen werden zum besseren Verständnis und zur Erzielung einer optimalen individuellen Therapie in Unterformen unterteilt, wobei die Übergänge der verschiedenen Unterformen oft fließend sind. Die Klassifizierung der Depression erfolgt - traditionell - nach ihren vermeintlichen Ursachen oder - neuerdings - nach ihren Symptomen (siehe hierzu ICD-10 "International Statistical Classification of Diseases and Related Health Problems" der WHO).

Unter dem Begriff "Depression" werden in dieser Patentanmeldung sowohl die verschiedenen, unten genannten traditionellen Unterformen der Depression verstanden, als auch die im ICD-10 unter dem Begriff "affektiven Störungen" subsumierten Störungen, die mit depressiven Episoden einhergehen, insbesondere depressive Episoden, rezidivierende depressive Störungen, depressive Phasen bei bipolaren affektiven Störungen sowie Angststörungen, Anpassungsstörungen und hirnorganische Erkrankungen, die jeweils mit depressiven Symptomen einhergehen. Entsprechende Störungen sind beispielsweise in den ICD-10 Klassifikationen (Version 2.0, Stand November 2000) F31, F32, F33, F41, F43, F45 und F06 aufgeführt.

Bei der traditionellen Unterteilung der Depression nach Ursachen werden üblicherweise 4 Hauptklassen unterschieden:

### I. Endogene Depressionen

Bei endogener Depression lassen sich keine ohne weiteres erkennbaren äußeren Ursachen als Auslöser der Depression identifizieren. Auslöser sind wahrscheinlich Störungen des Neurotransmittersystems des Gehirns. Typisch für endogene Depressionen ist der phasenhafte Verlauf, wobei die depressiven Episoden wiederholt auftreten können. Endogene Depressionen werden in der Regel unterteilt in
· unipolare Depressionen ("major depression"), bei der nur depressive Phasen auftreten
· bipolare Depressionen ("manisch-depressive Störungen"), bei denen depressive Episoden mit manischen Phasen wechseln.

### II. Somatogene Depressionen

Ursache dieser Depressionen sind körperlich-organische Störungen. Im Allgemeinen werden somatogene Depressionen unterteilt in
· organische Depressionen, die auf einer Erkrankung oder Verletzung des Gehirns beruhen. Solche Erkrankungen oder Verletzungen, die häufig mit einem veränderten Hirnstoffwechsel einhergehen, sind z.B. Hirntumore, Morbus Parkinson, Migräne, Epilepsie, Hirnlähmung, Hirnarteriosklerose, Hirntraumen, Hirnhautentzündung, Schlaganfall und Demenzen, wie z.B. die Alzheimersche Erkrankung;
·symptomatische Depression, die oft als Folge oder Begleiterscheinung einer Krankheit aufritt, die die Hirnfunktion nur indirekt beeinflusst. Dies kann z.B. eine Kreislauferkrankung, Hypothyreose oder eine andere Hormonstörung, Infektionskrankheit, Krebs oder Lebererkrankung sein;
· pharmakogene Depression, z.B. bei Alkohol-, Medikamenten- oder Drogenmissbrauch.

### III. Psychogene Depressionen

Diese sind oft Überreaktionen auf ein oder mehrere traumatische Erlebnisse. Die Unterteilung erfolgt häufig in Erschöpfungs-Depression, neurotische Depression und reaktive Depression auf Grund aktueller Konflikte oder Ereignisse.

### IV. Depressionen in besonderen Lebenslagen

Beispiele sind Wochenbett-Depressionen, Alters-Depressionen, Depressionen im Kindesalter, saisonale Depressionen sowie Pubertätsdepressionen.

Verbindungen der Formeln 1, insbesondere Rotigotin, sowie deren Salze sind grundsätzlich für die Herstellung eines Medikaments zur Behandlung der verschiedenen, oben genannten Depressionsformen bzw. zur Behandlung von affektiven Störungen, insbesondere von depressiven Episoden, rezidivierenden depressiven Störungen, Zyklothymia und von depressiven Phasen bei bipolaren affektiven Störungen, entsprechend der ICD-10 geeignet.

Die Verbindungen der Formeln I werden zur Herstellung eines Medikaments zur Behandlung depressiver Episoden und schwerer rezidivierender depressiver Störungen verwendet, wie sie beispielsweise bei der endogenen, unipolaren Depression ("major depression") auftreten.

Als Ursachen für endogene, unipolare Depressionen werden Stoffwechselstörungen der Gehirnzellen, d.h. Noradrenalin- oder Serotoninmangel und/oder eine genetische Prädisposition angesehen.

Unter dem Begriff "major depression" wird in dieser Patentanmeldung eine Störung umfasst, wie im amerikanischen Diagnose-Manual "The Diagnostic and Statistic Manual of Mental Disorders - 4th Edition" (American Psychiatric Association, 1994; "DSM IV") beschrieben.

Die Verbindungen der Formel I, insbesondere Rotigotine, und deren Salze sind auch besonders geeignet zur Herstellung von Antidepressiva zur Behandlung depressiver Episoden bei manisch-depressiven Patienten. Diese depressiven Phasen bei bipolaren Störungen werden in dieser Patentanmeldung unter dem Begriff "Depressionen" subsumiert.

Ferner werden die Verbindungen der Formeln I zur Herstellung eines Arzneimittels zur Behandlung "organischer" Depressionen verwendet die nicht in Zusammenhang mit Morbus Parkinson stehen wie weiter oben beschrieben. Organische Depressionen treten beispielsweise häufig bei Parkinson-Erkrankungen, bzw. bei zerebrovaskulären Erkrankungen und bei dementiellen Störungen auf.

Gegenstand der Erfindung ist die Verwendung von Verbindungen der Formel I, insbesondere Rotigotin, sowie Salze dieser Verbindungen, jeweils alleine oder in Kombination mit anderen Antidepressiva, zur Behandlung organischer Depressionen, die nicht im Zusammenhang mit Morbus Parkinson stehen. Beispiele für solche organischen Depressionen sind Depressionen im Zusammenhang mit Hirntumoren, Migräne, Epilepsie, Hirnlähmung, Hirnarteriosklerose, Hirntraumen, Hirnhautentzündung, Schlaganfall, Demenz, Alzheimer'sche Erkrankung oder dem Parkinson Plus Syndrom.

Verbindungen der Formeln 1 sind optisch aktiv und können als Razemate oder als reine (R)- oder (S)-Enantiomere vorliegen. Unter dem Begriff "reines- Enantiomer" wird in dieser Patentanmeldung verstanden, dass eine Substanz vorzugsweise zu mindestens 90 Mol%, besonders bevorzugt zu mindestens 95, 98 oder 99 Mol% in Form des einen Enantiomers, z.B. der (S)-Form, vorliegt, während der Anteil des jeweils anderen Enantiomers, z.B. der (R)-Form, entsprechend gering ist Wird zur Herstellung des erfindungsgemäßen Arzneimittels beispielsweise Rotigotin ([(-)-5,6,7,8-Tetrahydro-6-[propyl[2-(2-thienyl)ethyl]amino]-1-naphthol] verwendet, liegt das (R)-(+)-Enantiomer bevorzugt mit einem Anteil von < 10 Mol%, besonders bevorzugt mit einem Anteil von < 2 Mol% und ganz besonders bevorzugt mit einem Molanteil von < 1 % bezogen auf die Gesamtrotigotinmenge im Antidepressivum vor.

Verbindungen der Formel I können als freie Basen oder in Form der physiologisch akzeptablen Salze, z.B. in Form des Rotigotin-Hydrochlorids, im Arzneimittel vorliegen.

"Physiologisch akzeptable Salze" schließen nicht-toxische Additionssalze einer Base, insbesondere einer Verbindung der Formel I in Form der freien Base, mit organischen oder anorganischen Säuren, wie z.B. das HCl, ein.

Zur Verabreichung von Verbindungen der Formel 1 stehen verschiedene Applikationswege zur Verfügung, die der Fachmann je nach Bedarf, Zustand und Alter des Patienten, erforderlicher Dosierung und gewünschtem Applikationsintervall auswählen und anpassen kann.

Eine bevorzugte Art der Verabreichung von Verbindungen der Formel I ist die transdermale Gabe. Die Darreichungsform kann grundsätzlich ausgewählt sein aus z.B. Salbe, Paste, Spray, Folie, Pflaster oder einer iontophoretischen Vorrichtung.

Bevorzugt werden Verbindungen der Formel I, z.B. Rotigotin, in Pflasterform auf die Haut des Patienten gebracht, wobei der Wirkstoff bevorzugt in einer Matrix aus adhesivem Polymer, z.B. einem selbstklebenden adhesiven Polysiloxan, vorliegt (Ausführungsbeispiel 1) . Beispiele für geeignete transdermale Formulierungen finden sich in WO 99/49852, WO 02/89777 und WO 02/89778. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman,Clinical-Neuropharmacol. 24, 2001, 163).

Wird dagegen ein Antidepressivum in Form einer subkutanen oder intramuskulären Depotform gewünscht, kann eine Verbindung der Formel I beispielsweise als Salzkristall, z.B. als kristallines Hydrochlorid, in einem hydrophoben, wasserfreien Medium suspendiert und injiziert werden, wie in WO 02/15903 beschrieben oder auch in Form von Mikrokapseln, Mikropartikeln oder Implantaten auf Basis bioabbaubarer Polymere, wie beispielsweise in WO 02/38646 beschrieben, verabreicht werden.

Andere denkbare Formen der Verabreichung von Verbindungen der Formel I sind transmukosale Formulierungen, z.B. Sublingualsprays, rektale Formulierungen oder Aerosole zur pulmonalen Verabreichung.

Geeignete Dosierungen von Verbindungen der Formel I liegen im allgemeinen zwischen 0,1 und 50 mg/Tag, wobei vorzugsweise Tagesdosen zwischen 0,2 und 40 mg und insbesondere zwischen 0,4 und 20 mg/Tag verabreicht werden. Besonders bevorzugte Dosierungen von Verbindungen der Formel I, insbesondere von Rotigotin, liegen oberhalb von 0,5 mg/Tag, wobei für Anwendungen, die keine gleichzeitige Behandlung von motorischen Störungen von Morbus Parkinson erfordern, ganz besonders bevorzugt solche Dosierungsformen ausgewählt werden, in denen die antidepressive Wirkung von Verbindungen der Formel I, insbesondere von Rotigotin, ausgeprägt ist, bei denen die unspezifisch stimulatorische Wirkung von Verbindungen der Formel 1, insbesondere von Rotigotin, aber möglichst gering ist. Solche Dosierungen liegen im Allgemeinen unter 10 mg/Tag, z.B. unter 7,5 mg oder unter 5, 4, 3, 2 oder unter 1 mg/Tag und insbesondere zwischen 0,5 und 5 mg/Tag.

Bei Morbus Parkinson-Patienten kann dagegen eine Dosierung von z.T. oberhalb von 5 mg/Tag zur gleichzeitigen Therapie der motorischen Störungen erforderlich sein. Entsprechende Dosierungen liegen z.B. in Abhängigkeit von Alter und Verfassung des Patienten, Schweregrad der Erkrankung etc. zum Teil bei deutlich über 1 mg/Tag, z.B. bei über 5, 6, 7, 8, 9, 10 oder sogar zwischen 10 und 50 mg/Tag, z.B. zwischen 10 und 25 mg/Tag.

In Abhängigkeit von der gewählten Applikationsart kann die gewünschte Tagesdosis durch das Formulierungsdesign gesteuert werden. Beispielsweise kann die Tagesdosis von transdermal verabreichten Verbindungen der Formel I, insbesondere Rotigotin, durch die Einstellung einer entsprechenden Fluxrate pro Flächeneinheit und/oder durch Variation der Pflastergröße eingestellt werden. Dabei kann die Dosierung einschleichend erfolgen, das heißt, die Behandlung kann gegebenenfalls mit niedrigen Dosierungen beginnen, die dann bis zur Erhaltungsdosis gesteigert werden.

Ein Gegenstand der Erfindung ist daher eine Dosierungsform, z.B. ein Pflaster oder eine injizierbare Depotformulierung, die die entsprechende zur Therapie der Depression erforderliche Menge der Verbindung der Formel I, z.B. zwischen 0,5 und 10 mg/Tag oder zwischen 0,5 und 5 mg/Tag, wie weiter oben beschrieben, freisetzt.

Dem Fachmann ist klar, dass das Dosierungsintervall in Abhängigkeit von der applizierten Menge, der Applikationsart und dem Tagesbedarf des Patienten variieren kann. So kann eine transdermale Applikationsform beispielsweise zur einmal täglichen, dreitägigen oder siebentägigen Verabreichung konzipert sein, während ein subkutanes oder intramuskuläres Depot Injektionen beispielsweise im Ein-, Zwei- oder Vierwochen-Rhythmus ermöglichen kann.

Verbindungen der Formel I, insbesondere Rotigotin, können zur Monotherapie der Depression verwendet werden. In einer Ausführungsform der Erfindung können in der antidepressiven Arzneiform neben Verbindungen der Formel I aber auch noch andere Wirkstoffe vorliegen.

Beispiele hierfür sind andere Antidepressiva, die den Serotonin- oder Noradrenatin-Stoffwechsel direkt oder indirekt beeinflussen.

Beispiele hierfür sind
- selektive Serotonin-Wiederaufnahmehemmer wie Sertralin, Citalopram, Paroxetin oder Fluoxetin
- gemischte Serotonin-, Noradrenalin-Wiederaufnahmehemmer wie Venlaxafin, Milnacipram, Mirtazapin und trizyklische Antidepressiva wie Amitryptilin und Imipramin
- selektive Noradrenalin-Wiederaufnahmehemmer wie Reboxetin
- Monoaminoxidase-Hemmer wie Tranylcypramin oder Clorgylin
- Alpha2-Rezeptor und/oder Serotoninrezeptor-Modulatoren wie Mirtazapin oder Nefazodon.

Andere Beispiele für Antidepressiva sind Adenosin-Antagonisten, wie z.B. ST 1535, Sigma-Opioidrezeptor-Liganden, NK-Antagonisten wie GW 597599, Saredudant oder Aprepitant, Melatonin-Agonisten oder Modulatoren der Hypothalamus-Hypophyse-Nebennieren-Achse.

In Abhängigkeit von der Ursache und den Symptomen der Depression kann ein Kombinationspräparat auch ein zusätzliches Antipsychotikum, Sedativum, Anxiolytikum oder Migränemittel, bzw. einen Wirkstoff enthalten, der ein oder mehrere Wirkungen ausgewählt aus antidepressiver, antipsychotischer, sedativer, anxiolytischer oder antimigränoider Wirkung entfaltet.

Dabei können die Verbindung der Formel I und das zusätzliche Antidepressivum, Antipsychotikum, Sedativum, Anxiolytikum oder Migränemittel in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Beispiele für Antipsychotika sind Promethazin, Fluphenazin, Perphenacin, Levomepromazin, Thioridazin, Perazin, Promazin, Chlorprothixen, Zuclopenthixol, Prothipendyl, Flupentixol, Zotepin, Benperidol, Pipamperon, Melperon, Haloperidol, Bromperidol, Sulpirid, Clozapin, Pimozid, Risperidon, Quetiapin, Amisulprid, Olanzapin.

Beispiele für Sedativa sind Diphenhydramin, Doxylaminsuccinat, Nitrazepam, Midazolam, Lormetazepam, Flunitrazepam, Flurazepam, Oxazepam, Bromazepam, Triazolam, Brotizolam, Temazepam, Chloralhydrat, Zopiclon, Zolpidem, Tryptophan, Zaleplon.

Beispiele für Anxiolytika sind Fluspirilen, Thioridazin, Oxazepam, Alprazolam, Bromazepam, Lorazepam, Prazepam, Diazepam, Clobazam, Medazepam, Chlordiazepoxid, Dikaliumchlorazepat, Nordazepam, Meprobamat, Buspiron, Kavain, Hydroxyzin.

Beispiele für Migränemittel sind Almotriptan, Zolmitriptan, Acetylsalicylsäure, Ergotamin, Dihydroergotamin, Methysergid, Iprazochrom, Ibuprofen, Sumatriptan, Rizatriptan, Naratriptan, Paracetamol.

### Ausführungsbeispiele:

### Ausführungsbeispiel 1: Rotigotin-Pflaster

1.8 g Rotigotin (freie Base) werden in 2.4 g Ethanol gelöst und zu 0.4 g Kollidon 90F (gelöst in 1 g Ethanol) gegeben. Diese Mischung wird zu einer 74%igen Lösung von Silikonpolymeren (8.9 g BioPSA 7-4201 + 8.9 g BIO-PSA 7-4301 [Dow Corning]) in Heptan gegeben. Nach Zugabe von 2.65 g Petrolether wird die Mischung für 1 Stunde bei 700 UpM gerührt, um eine homogene Dispersion zu erhalten. Nach Laminierung auf Polyester wurde bei 50°C getrocknet. Das Pflastergewicht betrug schließlich 50 g/cm2.

### Ausführungsbeispiel 2: Rotigotin-Depotsuspensionen

(a) 1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.
(b) 1188 g der unter (a) hergestellten Lösung wurde in einen Glaslaborreaktor überführt, 12 g Rotigotin zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### Ausführungsbeispiel 3:

Die Bulbektomie-Studie wurde an Sprague-Dawley-Ratten durchgeführt. Als Kontrollgruppe diente eine scheinoperierte Gruppe, die operiert wurde, ohne dass die Riechkolben entfernt wurden. 14 Tage nach der Operation wurden die Ratten mit Vehikel, Rotigotin-Depotsuspension (jeden 2. Tag) oder Imipramin behandelt. An Testtagen wurden die Ratten auf ein Testfeld verbracht und für 3 Minuten sich selber überlassen. Dabei wurden die lokomotorischen Aktivitäten der Tiere anhand der Zahl überschrittener Linien gemessen.

## Patentansprüche

1. Verwendung einer Verbindung der allgemeinen Formel I 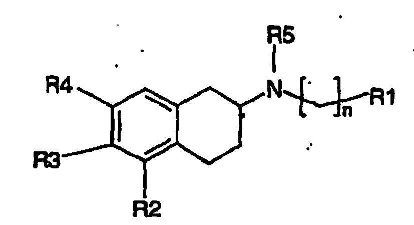 worin gift
n=1-5;
R2 ist = OA; R3 und R4 sind jeweils unabhängig voneinander ausgewählt aus H und OA; wobei A ausgewählt ist aus H, C1-3 Alkyl oder einer Gruppe, 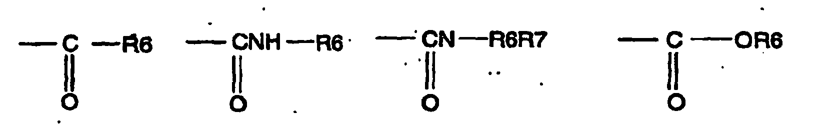 in der R6 und R7 unabhängig voneinander Alkyl oder Aryl sind;
R5 ist ein C1-3 Alkyl;
R1 ist eine Gruppe, die ausgewählt ist aus 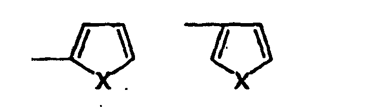 worin X ausgewählt ist aus S, O oder NH;
wobei die Verbindung der Formel I als Razemat oder als reines (R)- oder (S)-Enantiomer vorliegt;
sowie physiologisch akzeptable Salze dieser Verbindungen, zur Herstellung eines Arzneimittels zur Behandlung von endogenen oder von organischen Depressionen, die nicht im Zusammenhang mit Morbus Parkinson stehen.

2. Verwendung nach Anspruch 1, wobei R3 und R4 beide jeweils Wasserstoff darstellen.

3. Verwendung nach einem der vorhergehenden Ansprüche, wenn A ein Wasserstoffatom oder eine Gruppe 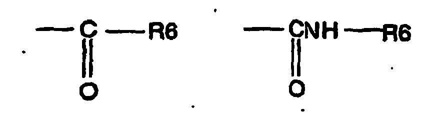 ist, in der R6 C1-12 Alkyl, Phenyl oder Methoxyphenyl ist.

4. Verwendung nach einem der vorhergehenden Ansprüche, wobei n den Wert 1-3 hat und R5 ein C3-Alkyl darstellt.

5. Verwendung nach einem der vorhergehenden Ansprüche, wobei X ein Schwefelatom ist.

6. Verwendung nach einem der vorhergehenden Ansprüche, wobei R1 ein 2-Thienyl ist.

7. Verwendung nach einem der vorhergehenden Ansprüche, wobei die Verbindung 5,6,7,8-Tetrahydro-6-[propyl[2-(2-thienyl)ethyl]amino]-1-naphthol ist.

8. Verwendung nach Anspruch 7, wobei die Verbindung das reine (S)-Enantiomer (Rotigotine) ist.

9. Verwendung nach einem der vorhergehenden Ansprüche, wobei die Depression eine unipolare Depression [major depression] oder eine depressive Phase einer manisch. depressiven Störung ist.

10. Verwendung nach einem der vorhergehenden Ansprüche, wobei die Depression eine von Morbus Parkinson unabhängige organische Depression ist.

11. Verwendung nach einem der vorhergehenden Ansprüche, wobei das Arzneimittel zur parenteralen, transdermalen oder mukosalen Administration vorgesehen ist

12. Verwendung nach einem der vorhergehenden Ansprüche, wobei die Verbindung der allgemeinen Formel I in einer Dosierung von 0,5 -50 mg pro Tag verabreicht wird.

13. Verwendung nach einen der Ansprüche 1-8, wobei das Arzneimittel ein Kombinationspräparat ist umfassend eine Verbindung nach einem der Ansprüche 1-8 und einen weiteren Wirkstoff aus der Gruppe der
- Antidepressiva, ausgewählt aus der Gruppe der selektiven Serotonln-Wiederaufnahmehemmer, der gemischten Serotonin-, Noradrenalin-Wiederaufnahmehemmer, der selektiven Noradrenalin-Wlederaufnahmehemmer, der Monoaminoxidase-Hemmer, der Alpha2-Rezeptor und/oder Serotonlnrezeptor-Modulatoren, der Adenosin-Antagonisten, der Sigma-Opioidrezeptor-Liganden, der NK-Antagonisten, der Melatonin-Agonisten oder der Modulatoren der. Hypothalamus-Hypophyse-Nebennieren-Achse;
- Antipsychotika, ausgewählt aus Promethazin, Fluphenazin, Perphenacin, Levomepromazin, Thioridazin, Perazin, Promazin, Chlorprothixen, Zuclopenthixol, Prothipendyl, Flupentixol, Zotepin, Benperidol, Pipamperon, Melperon, Haloperidol, Bromperidol, Sulpirid, Clozapin, Pimozid, Risperidon, Quetiapin, Amisulprid, Olanzapin;
- Sedativa, ausgewählt aus Diphenhydramin, Doxylaminsuccinat, Nitrazepam, Midazolam, Lormetazepam, Flunitrazepam, Flurazepam, Oxazepam, Bromazepam, Triazolam, Brotizolam, Temazepam, Chloralhydrat, Zopiclon, Zolpidem, Tryptophan, Zaleplon;
- Anxiolytika, ausgewählt aus Fluspirilen, Thioridazin, Oxazepam, Alprazolam, Bromazepam, Lorazepam, Prazepam, Diazepam, Clobazam, Medazepam, Chlordiazepoxid, Dikallumchlorazepat, Nordazepam, Meprobamat, Buspiron, Kavain, Hydroxyzin;
oder
- Migränemittel, ausgewählt aus Almotriptan, Zolmitriptan, Acetylsalicylsäure, Ergotamin, Dihydraergotamin, Methysergid, Iprazochrom, Ibuprofen, Sumatriptan. Rizatriptan, Naratriptan, Paracetamol,
ist.

## Claims

1. Use of a compound of the general formula I 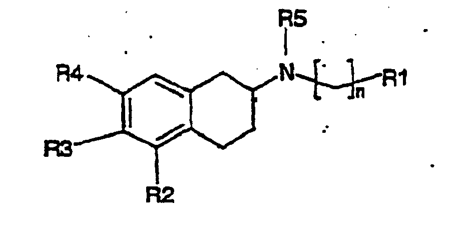 in which the following applies:
n = 1 - 5;
R2 = OA; R3 and R4 are each independently of one another selected from H and OA; wherein A is selected from H, C1-3 alkyl or a group 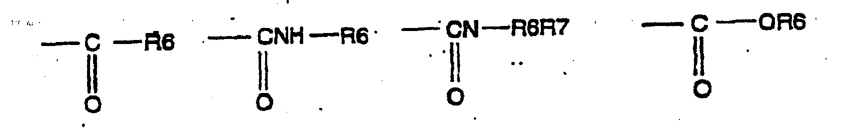 in which R6 and R7 independently of one another are alkyl or aryl;
R5 is a C1-3 alkyl;
R1 is a group which is selected from 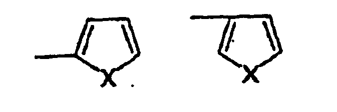 in which X is selected from S, O or NH;
wherein the compound of formula I is present as a racemate or a pure (R) or (S) enantiomer;
as well as physiologically acceptable salts of these compounds, for the manufacture of a medicament for the treatment of endogenous or of organic depressions which are not associated with Parkinson's disease.

2. Use according to claim 1, wherein both R3 and R4 each represent hydrogen.

3. Use according to one of the preceding claims, wherein A is a hydrogen atom or a group 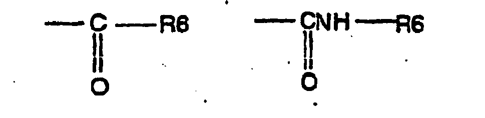 in which R6 is Cl-12 alkyl, phenyl or methoxyphenyl.

4. Use according to one of the preceding claims, wherein n has the value of 1-3 and R5 represents a C3 alkyl.

5. Use according to one of the preceding claims, wherein X is a sulfur atom.

6. Use according to one of the preceding claims, wherein R1 is a 2-thienyl.

7. Use according to one of the preceding claims, wherein the compound is 5,6,7,8-tetrahydro-6-[propyl[2-(2-thienyl)ethyl]amino]-1-naphthol.

8. Use according to claim 7, wherein the compound is the pure (S) enantiomer (rotigotine).

9. Use according to one of the preceding claims, wherein the depression is a unipolar depression (major depression) or a depressive phase of a manic-depressive disorder.

10. Use according to one of the preceding claims, wherein the depression is an organic depression independent of Parkinson's disease.

11. Use according to one of the preceding claims, wherein the medicament is intended for parenteral, transdermal or mucosal administration.

12. Use according to one of the preceding claims, wherein the compound of general formula I is administered in a dose of 0.5 - 50 mg per day.

13. Use according to one of cl ai ms 1 - 8, wherein the medicament is a combination preparation comprising a compound according to one of claims 1 - 8 and an additional active substance from the group of the
- antidepressants, selected from the group of the selective serotonin reuptake inhibitors, the mixed serotonin and noradrenalin reuptake inhibitors, the selective noradrenalin reuptake inhibitors, the monoaminoxidase inhibitors; the alpha-2 receptor and/or serotonin receptor modulators, the adenosine antagonists, the sigma opioid receptor ligands, the NK antagonists, the melatonin agonists or the modulators of the hypothalamic-pituitary-adrenal axis;
- antipsychotics, selected from promethazine, fluphenazine, perphenazine, levomepromazine, thioridazine, perazine, promazine, chlorprothixene, zuclopenthixol, prothipendyl, flupentixol, zotepine, benperidol, pipamperone, melperone, haloperidol, bromperidol, sulpiride, clozapine, pimozide, risperidone, quetiapine, amisulpride, olanzapine;
- sedatives, selected from diphenhydramine, doxylamine succinate, nitrazepam, midazolam, lormetazepam, flunitrazepam, flurazepam, oxazepam, bromazepam, triazolam, brotizolam, temazepam, chloral hydrate, zopiclone, zolpidem, tryptophan, zaleplon;
- anxiolytics, selected from fluspirilene, thioridazine, oxazepam, alprazolam, bromazepam, lorazepam, prazepam, diazepam, clobazam, medazepam, chlordiazepoxide, dipotassium clorazepate, nordazepam, meprobamate, buspirone, kavain, hydroxyzine;
or
- migraine agents, selected from almotriptan, zolmitriptan, acetylsalicylic acid, ergotamine, dihydroergotamine, methysergide, iprazochrome, ibuprofen, sumatriptan, rizatriptan, naratriptan, paracetamol.

## Revendications

1. Utilisation d'un composé de formule générale I 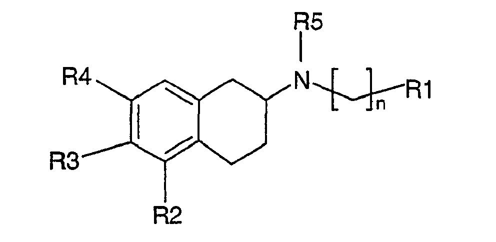 dans laquelle :
n = 1 - 5 ;
R2 est = OA ; R3 et R4 sont, chacun indépendamment, choisis parmi H et OA ; où A est choisi parmi H, alkyle en C₁₋₃ ou un groupe : 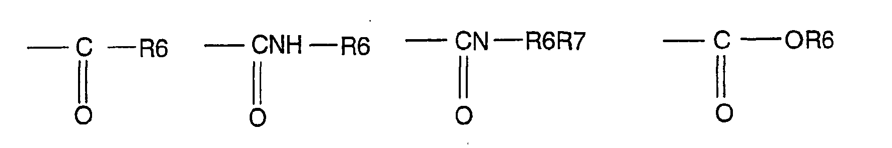 dans lesquels R6 et R7, indépendamment, sont des alkyles ou des aryles ;
R5 est un alkyle en C₁₋₃ ;
R1 est un groupe qui est choisi parmi 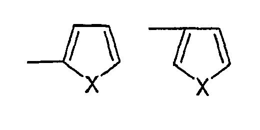 où X est choisi parmi S, O ou NH ;
où le composé de formule I se présente sous forme racémique ou sous forme d'énantiomère pur (R) ou (S) ;
et les sels physiologiquement acceptables de ces composés, pour la fabrication d'un médicament pour le traitement des dépressions endogènes ou organiques, qui ne sont pas en relation avec la maladie de Parkinson.

2. Utilisation selon la revendication 1, où R3 et R4 représentent tous deux respectivement l'hydrogène.

3. Utilisation selon l'une des revendications précédentes, dans laquelle A est un atome d'hydrogène ou un groupe 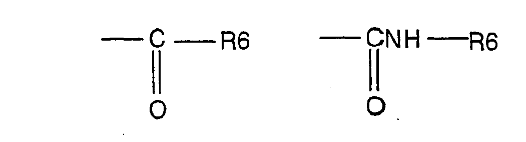 où R6 est un alkyle en C₁₋₁₂, un phényle ou un méthoxyphényle.

4. Utilisation selon l'une des revendications précédentes, où n prend la valeur 1 - 3 et R5 représente un alkyle en C₃.

5. Utilisation selon l'une des revendications précédentes, où X est un atome de soufre.

6. Utilisation selon l'une des revendications précédentes, où R1 est un 2-thiényle.

7. Utilisation selon l'une des revendications précédentes, où le composé est le 5,6,7,8-tétrahydro-6-[propyl-[2-(2-thiényl)éthyl]amino]-1-napthol.

8. Utilisation selon la revendication 7, où le composé est l'énantiomère (S) pur (rotigotine).

9. Utilisation selon l'une des revendications précédentes, où la dépression est une dépression unipolaire (dépression majeure) ou une phase dépressive d'un trouble maniaco-dépressif.

10. Utilisation selon l'une des revendications précédentes, où la dépression est une dépression organique indépendante de la maladie de Parkinson.

11. Utilisation selon l'une des revendications précédentes, où le médicament est destiné à l'administration parentérale, transdermique ou mucosale.

12. Utilisation selon l'une des revendications précédentes, où le composé de formule générale I est administré en un dosage de 0,5 à 50 mg par jour.

13. Utilisation selon l'une des revendications 1 à 8, où le médicament est une préparation combinée comprenant un composé selon l'une des revendications 1 à 8 et un autre principe actif du groupe des :
- antidépresseurs, choisis dans le groupe des inhibiteurs sélectifs de la réabsorption de la sérotonine, des inhibiteurs mixtes de la réabsorption de la sérotonine, noradrénaline, des inhibiteurs sélectifs de la réabsorption de la noradrénaline, des inhibiteurs de la monoaminoxydase, des modulateurs du récepteur alpha2 et/ou du récepteur de la sérotonine, des antagonistes de l'adénosine, des ligands du récepteur sigma-opioïde, des antagonistes NK, des agonistes de la mélatonine ou des modulateurs de l' axe hypothalamus - hypophyse - surrénales ;
- antipsychotiques, choisis parmi la prométhazine, fluphénazine, la perphénacine, la lévomepromazine, la thioridazine, la pérazine, la promazine, le chlorprothixène, le zuclopenthixol, le prothipendyle, le flupentixol, la zotépine, le benpéridol, le pipampéron, le melpéron, l'halopéridol, le brompéridol, le sulpiride, la clozapine, le pimozide, le rispéridon, la quétiapine, l'amisulpride, l'olanzapine ;
- sédatifs, choisis parmi la diphènhydramine, le succinate de doxylamine, le nitrazépam, le midazolam, le lormétazépam, le flunitrazépam, le flurazépam, l'oxazépam, le bromazépam, le triazolam, le brotizolam, le ternazépam, l'hydrate de chloral, le zopiclon, le zolpidem - tryptophane, le zalépion ;
- anxiolytiques, choisis parmi le fluspirilène, la thioridazine, l'oxazépam, l'alprazolam, le bromazépam, le lorazépam, le prazépam, le diazépam, le clobazam, le médazépam, le chlordiazépoxyde, le chlorazépate dipotassique, le nordazépam, le meprobamate, le buspiron, la kavaïne, l'hydroxyzine ;
ou
- antimigraineux, choisis parmi l'almotriptane, le zolmitriptane, l'acide acétylsalicylique, l'ergotamine, la dihydroergotamine, le méthysergide, l'iprazochrome, l'ibuprofène, le sumatriptane, le rizatriptane, le naratriptane, le paracétamol.
